# EUROPÄISCHE PATENTSCHRIFT

(11) **EP 0 891 977 B1**
(45) Veröffentlichungstag und Bekanntmachung des Hinweises auf die Patenterteilung: **29.01.2003**
(21) Anmeldenummer: 98112923.2
(22) Anmeldetag: 11.07.1998
(51) Int. Cl.: C07D 487/22, A01N 25/10, A61K 31/40, A61K 31/555, A01N 43/90, A01N 55/02, C02F 1/00

(54) **5-(4-Hydroxyphenyl)-porphyrinderivate als Photosensibilisatoren**
5-(4-Hydroxyphenyl)-porphyrin derivatives as photosensitisers
Dérivés de la 5-(4-hydroxyphényl)-porphyrine comme photosensibilisateurs

(30) Priorität: 16.07.1997 DE 19730469; 04.10.1997 DE 19743903
(43) Veröffentlichungstag der Anmeldung: 20.01.1999
(73) Patentinhaber: DLR Deutsches Zentrum für Luft- und Raumfahrt e.V., 51147 Köln (DE)
(72) Erfinder: Funken, Karl-Heinz, Dr. rer. nat., 53225 Bonn (DE); Ortner, Jürgen, Dr. rer. nat., 51065 Köln (DE); Braun-Milow, Barbara, Dr., 50354 Hürth (DE); Faust, Delia, 53783 Eitorf (DE); Horneck, Gerda, Dr., 56170 Bendorf (DE); Schäfer, Manfred, Dr., 63607 Wächtersbach (DE); Schmitz, Claudia, 50226 Frechen (DE); Sattlegger, Michael, Dr., 53121 Bonn (DE)
(74) Vertreter: Jönsson, Hans-Peter, Dr.Dipl.-Chem.

(56) Entgegenhaltungen:
- EP-A- 0 127 797
- EP-A- 0 345 171
- WO-A-93/00815
- CHEMICAL ABSTRACTS, vol. 122, no. 12, 20. März 1995 Columbus, Ohio, US; abstract no. 141379e, T.A.LEWIS ET AL : "Reductive declhorination of carbon tetrachloride mediated by cationic water-soluble metalloporphyrins" Seite 574; XP002081841 & J.ENVIRON.QUAL., Bd. 24, Nr. 1, 1995, Seiten 56-61,
- CHEMICAL ABSTRACTS, vol. 107, no. 107, 30. November 1987 Columbus, Ohio, US; abstract no. 211088a, H. ISHII ET AL : "Spectrophotometric and analog derivative spectrophotometric determination of trace amounts of iron using sulfonated 5-(3,4-dihydroxyphenyl)-10,15,20-triphenyl porphine" Seite 879; XP002081842 & ANALYST, Bd. 112, Nr. 8, 1987, Seiten 1121-1126,
- CHEMICAL ABSTRACTS, vol. 114, no. 20, 20. Mai 1991 Columbus, Ohio, US; abstract no. 198379u, L. DING ET AL : "Synthesis of water-soluble, cationic functionalized metalloporphyrins having a cytotoxic activity" Seite 888; XP002081843 & NEW J. CHEM., Bd. 14, Nr. 6-7, 1990, Seiten 421-431,

## Beschreibung

Gegenstand der Erfindung sind metallierte oder unmetallierte Porphyrinderivate sowie ein Verfahren zu ihrer Herstellung sowie deren Verwendung. Weiterhin sind Gegenstand der vorliegenden Erfindung Photosensibilisatoren umfassend die genannten Porphyrinderivate soweit die Verwendung der Photosensibilisatoren zur Initiierung photochemischer Reaktionen, insbesondere zur katalytischen Bildung von Singulett-Sauerstoff.

Die Erfindung betrifft insbesondere oberflächengeladene Porphyrinderivate, welche kovalent an einen polymeren Werkstoff gebunden sind.

Hydrophile polymergebundene Porphyrine besitzen vorteilhafte Eigenschaften als Photosensibilisatoren. Diese Vorteile kommen insbesondere an einer Grenzschicht Polymer - Wasser zur Geltung, da mit diesen neuartigen Stoffen auch ansonsten hydrophobe Polymeroberflächen in einen ausreichend intensiven Kontakt zu einer wäßrigen Phase gebracht werden können. So lassen sich photochemische Reaktionen initiieren, die ansonsten nicht ablaufen würden. Für die wasserlöslichen polymergebundenen Porphyrine gibt es potentiell andere Anwendungsbiete bzw. Betriebsweisen als für die wasserunlöslichen hydrophilen Polymere.

Unmetallierte Porphyrine und auch metallierte Porphyrine können als effiziente Photosensibilisatoren genutzt werden. Sie werden bei der Bestrahlung mit Licht ausreichender Wellenlänge elektronisch angeregt. In geeigneter Kombination mit chemischen Reaktionspartnern können die angeregten Sensibilisatoren entweder die Energie oder eine Ladung auf den betreffenden Reaktionspartner übertragen, welcher in nachfolgenden Reaktionen abreagiert. Als Akzeptor der Energie kommt eine Vielzahl anderer Spezies in Frage. Auch können elektronisch angeregte Porphyrine sowohl als Donoren wie auch als Akzeptoren unter Bildung von Exciplexen fungieren, die dann zu chemischen Reaktionen führen können (T. Mashiko, D. Dolphin, Comprehensive Coord. Chem. 2, 1987, 813-898).

Von anwendungstechnischem Interesse sind z.B. sensibilisierte Photooxygenierungen, sensibilisierte Photoisomerisierungen, sensibilisierte Photocycloadditionen und sensibilisierte Photodesulfonylierungen. Die sensibilisierte Aktivierung von Sauerstoff, der im Grundzustand als Triplett-Sauerstoff vorliegt und sensibilisiert in Singulett-Sauerstoff überführt werden kann, ist besonders bedeutsam. Singulett-Sauerstoff besitzt eine vom Triplett-Sauerstoff verschiedene Reaktivität. In vielen praktischen Fällen wird der Sensibilisator im Reaktionsgemisch homogen gelöst.

Kationische wasserlösliche Metalloporphyrine sind zum Beispiel für die reduktive Dechlorierung von Tetrachlorkohlenstoff beschrieben worden, insbesondere als Ersatz für den Abbau halogenierter Schmutzstoffe durch Mikroben in Abwässern (T. A. Louis et al. J. Environ. Qual. 1995 24 56-61).

Weitere kationische wasserlösliche Metalloporphyrinderivate werden beispielsweise in der EP 0 345 171 A1, wasserlösliche anionische Porphyrinderivate werden in der EP 0 127 797 A1 beschrieben.

Dabei ist es jedoch von Nachteil, daß der Sensibilisator nach erfolgter Reaktion bei der Aufarbeitung des Reaktionsgemischs häufig mit hohem Aufwand abgetrennt und ggf. recycliert werden muß. Außerdem wird oft gefordert, daß das Produkt keine Sensibilisatorreste mehr enthalten darf. Ein an sich bekannter Weg zur Verminderung des Aufwandes besteht darin, den Sensibilisator an Oberflächen von Trägermaterialien entweder physikalisch (durch Adsorption) oder chemisch (durch kovalente, koordinative oder elektrostatische Bindung) zu fixieren. Er kann dann nach erfolgter Reaktion z.B. durch Filtration leicht abgetrennt und wiederverwendet werden. In der Literatur werden Beispiele zur Verwendung von Sensibilisatoren und zu ihrer Heterogenisierung beschrieben (A.M. Braun, M.-T. Maurette, E. Oliveros, Photochemical Technology, John Wiley & Sons, Chichester 1991; H. Böttcher (Ed.), Technical Applications of Photochemistry, Deutscher Verlag für Grundstoffindustrie 1991).

Die Adsorption von Rose Bengale an Kieselgel beschreiben S. Tamagaki et al. (J. Org. Chem. 45, 1573, 1980).

Die Salzbildung ist unter anderem von D. Brkic et al. (J. Mol. Catal. 3, 173, 1977/78) für Rose Bengale an Kieselgel und von F. Le Guern et al. (Bull. Soc. Chem. Fr. 130, 753, 1993) für Zinn-Porphyrine an Kieselgel und Zeolithe beschrieben.

A.P. Shaap et al. (J. Am. Chem. Soc. 97, 3741, 1975) beschreiben die Umsetzung von Merrifield-Harz, einem quervemetzten Copolymer aus Chlormethylstyrol und Divinylbenzol, mit Rose Bengale. Dabei werden Estergruppen zwischen dem Polymer und dem Farbstoff ausgebildet. Das entstandene photosensitive Polymer ist hydrophob und schwer löslich in den meisten organischen Lösungsmitteln. Die Steigerung der Hydrophilie dieser Polymergruppe ist beschrieben von A.P. Shaap et al. (J. Am. Chem. Soc. 101, 4016, 1979). Die Verwendung von Ethylenglycolmethacrylester und Ethylglycoldimethylacrylester als zweite Komponente bei der Copolymerisation von Chlormethylstyrol liefert hydrophilere Polymere.

D. Wöhrle et al. (Makromol. Chem. 192, 819, 1991) berichten über ein Polymer mit kovalent gebundenem Zink-tetraphenylporphyrin. Zum Einsatz kommt Zink-5-(4-aminophenyl)-10,15,20-triphenylporphyrin. Dieses wird einerseits an Polyvinylchormethylstyrol angebunden und andererseits an aktiviertes Polymethacrylat als Amid. Auch ein Copolymer aus Poly(N-vinyl-2-pyrrolidon)-Methacrylsäure wurde verwendet, bei dem nach der Amidbildung die freien Carbonsäuregruppen methyliert wurden.

Heuermann et al. (Photodynamic Therapy and Biomedical Lasers 855, 1992) berichten über die Anbindung von metallierten 5,10,15,20-Tetrahydroxyphenyl-, Tetraaminophenyl, Tetracarboxyphenylporphyrinen an Methoxypolyethylen.

In WO 93/00815) wird die physikalische Bindung von unmetallierten Porphyrinen an Polymere wie Cellulose und deren Derivate sowie an Polyolefine und olefinische Polymere beschrieben. Zur Darstellung der photosensitiven Polymeren wird ein Extraktionsverfahren und das aus der Schmelzeziehen genannt. Es wird die Darstellung von photosensitiven Polymeren aus Farbstoffmonomeren und einem Copolymer beschrieben. Dabei werden unmetallierte Porphyrine verwendet. Die Monomere werden durch die Reaktion von Porphyrinen, die eine Alkoholgruppe besitzen, und Methacrylsäurechlorid gewonnen. Das zweite Monomer ist Methacrylsäuremethylester. Das Ergebnis ist ein modifiziertes Polymethylmethacrylat, das den Farbstoff über eine Esterfunktion gebunden hat. Auch das reine Polymer mit dem Farbstoff als Alkoholkomponente der Esterfunktion ist beschrieben. Als weiteres farbstofftragendes Monomer wird Protoporphyrindimethylester verwendet, das mit Methacrylsäuremethylester copolymerisiert wird.

H. Kamogawa et al. (J. Polymer Sci. 12, 2317, 1974) berichten über die Darstellung von Monomeren aus Vinylbenzylester mit metallierten und unmetallierten Protoporphyrin aus Chlormethylstyrol und dem Protoporphyrin. Dieses Monomer wurde copolymerisiert mit N-Vinylpyrrolidon, um zu wasserlöslichen Polymeren zu gelangen. Hier wird die Umsetzung eines Copolymers aus Chlormethylstyrol und N-Vinylpyrrolidin mit metallierten und unmetallierten Porphyrinen beschrieben. Als verbindende Gruppe zwischen dem Photosensibilisator und dem Polymer wird hierbei einmal eine Estergruppe gebildet und zum anderen eine Schiffsche Base. Als Farbstoffe werden zum einen Porphyrine mit Carbonsäuregruppen und zum anderen Aldehydgruppen verwendet.

M. Kamachi et al. (Macromolecules 20, 2665, 1987) beschreiben in einem zweiten Artikel die Copolymerisation von unmetalliertem 5-(4-Acryloyloxyphenyl)-10,15,20-triphenylporphyrin und 5-(4-Methyacryloyloxyphenyl)-10,15,20-triphenylporphyrin mit Methacrylsäuremethylester. Das mit Eisen, Vanadium oder Kobalt metallierte Polymer wurde durch nachträgliche Metallierung oder durch den Einsatz von metallierten Monomeren gewonnen (Chem. Letters 2331, 1987).

M. Kamachi et al. (J. Polymer Sci. 21, 693, 1983) beschreibt die Polymersiation von 5-(4-Acryloyloxyphenyl)-10,15,20-triphenylporphyrin. Auch der Einsatz des metallierten Monomers ist beschrieben worden. Das gewonnene Polymer ist unlöslich in Ethanol, Aceton, Wasser und Hexan. Das unmetallierte Polymer konnte mit Kupfer nachträglich metalliert werden.

H. Eichhorn et al. (Macromol. Chem. Phys. 196, 115, 1995) beschreiben die Darstellung von Monomeren basierend auf unmetalliertem 5,10,15,20-Tetraphenylprophyrin. Doppelbindungshaltige Gruppen wurden in den vier para-Positionen der Phenylringe eingefügt. So wurde über die Phenolgruppe Methacryloylreste angebunden. Bei der Copolymerisation mit Styrol wurden Polymere erhalten, die das Porphyrin als Knotenpunkt beinhalten.

D.H. Grayson et al. (Sol. Chem. R&D Eur. Community, Ser. D., 2, 51, 1983) beschreiben ein Polymer aus polyvinylpyridingebundenem Zinktetraphenylporphyrin.

W. Wolters beschreibt in DE 3836759 A1 und DE 3924815 A1 die Verwendung von Phthalocyaninen und diazotiertem β-Naphthol, physikalisch gebunden, das mit einem Bindemittel als Katalysator zur Aktivierung von Sauerstoff fixiert ist. Als Bindemittel werden einerseits Acrylsäureester und/oder Methacrylsäureester und andererseits Polyurethane verwendet.

Im Stand der Technik sind eine Reihe von Verfahren zur Desinfektion und/oder Detoxifikation von Wasser und/oder Wasser enthaltenen Umgebungen sowie von Gegenständen bekannt.

An erster Stelle sei hier bekanntermaßen die Chlorierung genannt. Siehe hierzu beispielsweise Dugan A.M. 1996, The products of water chlorination as inducers of gene mutations, Tsitol. Genet. 30: 76 - 81; Craun, G.F. 1988, Surface water supplies and health, J. Am. Water Works Assoc.: 80: 40 - 42; Singh, A. Yeager R., and McFeters G.A. 1986a, Assessment of in vivo revival, growth and pathogenicity of E. coli strains after copper- and chlorine-induced injury, Appl. Environ. Microbiol. 52: 832 - 837; Zierler, S., Danly R.A., Feingold L., 1986, Type of disinfectant in drinking water and patterns of mortality in Massachusetts, Environ. Health Perspect 69: 275 - 279; Jolley, R.L., Brungs W.A., and Cummings R.B., Eds. 1985, Water chlorination: Chemistry, Environmental Impact, and Health Effects, Lewis Pubs., Chelsea, Ml; Tuthill, R. W., Giusti R.A., Moore G.S., Calabrese E. J. 1982, Health effects among newborns after prenatal exposure to ClO₂-disinfected drinking water. Environ. Health Perspect 46: 39- 45. Der wesentliche Nachteil der Chlorierung besteht in den hohen Kosten durch permanenten Chemikalienverbrauch. Darüber hinaus werden bekanntermaßen giftige, beispielsweise kanzerogene oder mutagene Nebenprodukte, wie Trihalomethane und andere organische chemische Verbindungen gebildet. Weiterhin beeinträchtigt der unangenehme Geruch und der Geschmack das Wasser. Bekanntermaßen sind einige Mikroorganismen resistent gegen Chlor, so daß häufig noch eine ergänzende Behandlung angeschlossen werden muß. Viele Mikroorganismen werden durch Chlor zwar in ihrer Reproduktionsfähigkeit gehindert, sind jedoch trotzdem in der Lage, Enterotoxine zu produzieren.

Neben der Chlorierung ist die Chloraminierung, beispielsweise aus Sobsey, M.D. 1989, Inactivation of health-related microorganisms in water by disinfection processes, Water Sci. Technol. 21: 179 - 195; Groethe, D. R. und Eaton J. G. 1975, Chlorine induced mortality in fish. Trans. Am Fish Soc. 104: 800 - 805 bekannt. Auch hier besteht ein wesentlicher Nachteil in den hohen Kosten durch permanenten Chemikalienverbrauch. Wenn auch hier keine Trihalomethane gebildet werden, so ist doch die Chloraminierung nicht so effektiv wie die Chlorierung. Ein unangenehmer Geschmack ist auch hier bei der Detoxifizierung und Desinfektion von Wasser zu beobachten. Viele Organismen sind resistent gegen die Chloranimierung, so daß auch hier zusätzliche Maßnahmen erforderlich sind. Besonders hervorzuheben ist, daß die Chloranimierung sehr schädlich für aquatische Systeme ist. Mutagene und kanzerogene Effekte auf den Menschen werden vermutet.

Die Wasserbehandlung mit Chlordioxid ist beispielsweise aus Berg, J.D., Roberts P.V., and Martin A. 1986, Effect of chlorine dioxide an selected Membrane functions of Escherischia coli. J. Appl. Bacteriol. 60: 213 - 220 bekannt. Chlordioxid muß vor Gebrauch stets neu gebildet werden, da es keine Möglichkeit der Lagerung gibt. Dementsprechend ist das Verfahren wesentlich teurer als die Behandlung des Wassers mit Chlor. Eine Bildung von Trihalomethanen und Chloramin ist nicht zu befürchten, jedoch führen die Nebenprodukte Chlorit und Chlorat zur Methämoglobinämie, da sich diese auf das Hämoglobin des Menschen auswirken können.

Die Ozonisierung gemäß Kir'ianova, E.V. 1996, Optimization of conditions of ozone water disinfection by the method of mathematical planning of an experiment, Gig. Sanit. 4: 3-6; Farooq, S., Akhlaque S. 1983, Comparative response of mixed cultures of bacteria and viruses to ozonisation. Water Res. 17: 809 - 812 ist in Bezug auf die Konstruktion, den Betrieb und die Wartung sehr aufwendig. Da Ozon keine stabile Verbindung ist, muß das Wasser zur längeren Haltbarkeit meist noch mit Chlor versetzt werden. Auch gegen Ozon sind einige Mikroorganismen resistent. Als Nebenprodukte bilden sich häufig Aldehyde, die mutagen wirken können.

Ein weiteres Verfahren zur Behandlung von Wasser und/oder Wasser enthaltenen Umgebungen besteht in der Bestrahlung mit UV-Licht. Siehe hierzu beispielsweise Harris, M. G., Buttino L.M., Chang J.C., Wan M. 1993, Effects of ultraviolet radiation on contact lense parameters , Optom. Vis. Sci 70: 739 - 742; Martiny, H., Wlodavezyk K., Harms G., Ruden H. 1988, The use of UV rays for the disinfection of water. I. Microbiologic studies of drinking water. Zentralbl. Bakteriol. Mikrobiol. Hag. (B). 185: 350- 367; Harris, G.V., Adams V.D., Sorensen D.L., and Dupont R.R. 1987, The influence of photoreactivation and water quality on ultraviolet disinfection of secondary municipal wastewater. J. Water Pollut. Control Fed. 59: 781 - 787; Muraca, P., Stout J.E., Yu V.L. 1987, Comparative assessment of chlorine, heat, ozone, an UV-light for killing Legionella pneumophila within a model plumbing system, Appl. Environ. Microbiol. 53: 447 - 453; Myhrstadt, J.A., 1979, Disinfection of sewage water by ultraviolet irradiation. NIPH, Ann 2: 11 - 16. Da hier keine aktiven Substanzen in behandeltem Wasser verbleiben, muß in der Regel nachträglich chloriert werden, wodurch wiederum zusätzliche Kosten entstehen. In Abhängigkeit von der Trübung, dem Sauerstoffgehalt, dem Anteil und der Art gelöster Teilchen und chemischer Verunreinigungen des zu behandelnden Wassers ist die Dosisbestimmung sehr problematisch. Es besteht die Möglichkeit der Photoreaktivierung; es muß daher sichergestellt werden, daß das Wasser nicht während oder nach der UV-Bestrahlung an Licht aufbewahrt wird.

Durch Biofilmbildung auf der Lampe ist eine ständige Überwachung und sorgfältige Reinigung der Lampe - chemisch, mechanisch oder mit Ultraschall - wichtig, wodurch zusätzliche Wartungskosten entstehen. Beim Betrieb der Lampe ist auf einen ausreichenden Personenschutz zu achten, da das UV-Licht bekanntermaßen mutagen und kanzerogen wirkt. Durch UV-Licht ermüden eine Reihe von Materialien. Bekanntermaßen wird durch UV-Licht die Bildung von gesundheitsschädlichem Ozon gefördert.

Aus Ahlstrom, S.B. and Lessel T., 1986, Irradiation of municipal sludge for pathogen control. In: Control of Sludge Pathogens, C.A. Sorber, Ed. Water Pollution Control Fedration, Washington D.C. ist die Behandlung von Wasser mit GammaStrahlung und hochenergetischer Elektronenstrahlung bekannt. Hierbei handelt es sich um sehr teure aufwendige Anlagen, die ein hohes Sicherheitsrisiko beinhalten. Die Wirkung kann verbessert werden, wenn zusätzlich noch Hitze oder Sauerstoff zugeführt wird, wodurch sich jedoch die Kosten weiterhin erhöhen.

Aus Polyzois, G.L., Zissis A. J., Yannikakis S.A. 1995. The effect of glutaraldehyde and microwave disinfection on some properties of denture resin. Int. J. Prosthodont 8: 150 - 154; und Niederwohrmeier, B., Bohm R., Strauch D. 1985, Microwave treatment as an alternative pasteurization process for the disinfection of sewage sludge: Experiments with the treatment of liquid manure, pp. 135-147 in: Inactivation of Microorganisms in Sewage Sludge by Stabilisation Processes, D. Strauch, A.H. Havelaar, and O.L. L'Hermite, Eds. Elsevier Applied Science, London ist die Behandlung von Wasser mit Mikrowellenstrahlung bekannt. Auch diese Maßnahme ist sehr teuer, da aufwendige Anlagen und Sicherheitsmaßnahmen erforderlich sind. Durch die zusätzliche Wärmezufuhr werden die Kosten weiterhin erhöht, wobei jedoch die Wirkung der Strahlung verbessert ist. Für große Volumina ist die Mikrowellenstrahlung jedoch sehr problematisch, da eine optimale Durchmischung gewährleistet sein sollte.

Aus Joyce, T.M., McGuigan K.G., Elmore-Meegan M., Conroy R.M. Inactivation of fecal bacteria in drinking water by solar heating, Appl. Environ. Microbiol. 62: 399 - 402 und Fuhrmann, H., Floerke I., Bohm K.H. 1986, The problem of heat activation of bacterial spores after disinfection with regard to an aerosol method of decontaminating equipment and rooms. Zentralbl. Bakteriol. Mikrobiol. Hyg. (B) 182: 515 - 524 ist die Hitzesterilisation bekannt. Auch diese ist mit einem hohen Energieaufwand verbunden, wobei nicht immer eine vollständige Inaktivierung aller Organismen erreicht wird. Auch ist die Hitzesterilisation nicht für große Mengen geeignet.

Hegna, I.K., Clausen O.G. 1988, An investigation of the bactericidal and fungicidal effects of certain disinfectants by use of capacity fest. Ann. Inst. Pasteur., Microbiol. 139: 473 - 483; LeChevallier, M.W., and McFeters G.A. 1985b. Ennumerating injured coliforms in drinking water, J.Am. Water Works Assoc. 77: 81 87; Conn, H., Langer R. 1981, Iodine disinfection of hydrophilic contact lenses. Ann. Ophthalmol. 13: 361 - 364 beschreiben anderweitige Zusätze von Chemikalien und Metallen. Auch diese Verfahren sind durch ständigen Substanzverbrauch, durch aufwendige Rückgewinnung der Substanzen, durch eine Gesundheitsschädigung durch die zugesetzten Substanzen, beispielsweise Formaldehyd, Phenol und Glutaraldehyd gekennzeichnet. In vielen Fällen ist eine Gesundheitsschädigung durch entstehende Nebenprodukte nicht auszuschließen.

Archer, A., Fischer E., Zellingher R., Manor Y. 1990, Photochemical disinfection of effluents-pilot plant studies. Wat. Res. 24: 837 - 843; Dahl, T.A., Midden W. R., Neckers D.C. 1988; Comparison of photodynamic action by Rose Bengal in gram-positive and gram-negative bacteria. Photochem. Photobiol. Vol. 48 No. 5: 607 - 612; Archer, A. J., Juven B. J. 1976, Destruction of Coliforms in Water and Sewage Water by Dye-Sensitized Photooxidation Appl. Environ. Microbiol. 33: 1019 - 1022 beschreiben die photodynamische Desinfektion mit in Wasser gelösten Farbstoffen. Auch hier ist ein ständiger Substanzverbrauch erforderlich sowie eine Schwierigkeit der Rückgewinnung der Substanzen, beispielsweise Methylenblau oder Bengal Rosa, zu verzeichnen.

Die Filtration, die beispielsweise von Dorau, W. 1996, Mikrofiltration als Schlußreinigung bei der Abwasserreinigung, Notwendigkeit , verfahrenstechnische Einbindung, Kosten, Gewässerschutz-Wasser-Abwasser 152: 51/1-28 und Kolega, M., Grohmann G.S., Chiew R.F., Day A.W. 1991, Disinfection and clarification of treated sewage by advanced micorfiltration. Water Sci. Technol. 23: 1609 - 1618 beschrieben wird, ist eine aufwendige Maßnahme zur Behandlung von Wasser. Die Filter verstopfen sehr leicht, so daß ein hoher Wartungsaufwand erforderlich ist, der eine regelmäßige mechanische und chemische Reinigung der Anlage bedingt.

Die bekannten photosensitiven Polymere auf Basis von Polymethylmethacrylat sind bisher entweder wasserlöslich oder unlöslich. Ein Polymer mit hydrophilem Charakter, das jedoch wasserunlöslich ist, ist nicht bekannt. Die auf der Basis von metallierten und unmetallierten Tetraphenylporphyrin gebundenen Porphyrine in den bekannten Polymeren besitzen keine Gruppen in para-Stellung der Phenylgruppen, die eine positive oder negative Ladung tragen. Der Zugang zu Polymeren auf Basis von Polymethylmethacrylat und Tetraphenylporphyrinen ist nur über die Monomere beschrieben worden.

Die vorgenannte Aufgabe der vorliegenden Erfindung wird in einer ersten Ausführungsform gelöst durch metallierte oder unmetallierte Porphyrinderivate der allgemeinen Formel I 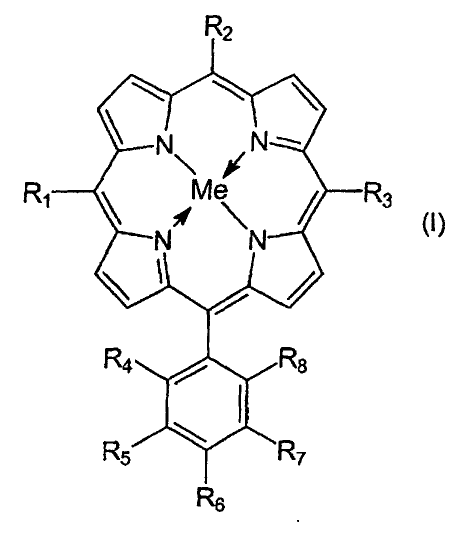 wobei
R₁, R₂ und R₃ jeweils für Wasserstoff, Alkyl, Aryl, Aralkyl oder Alkaryl, Sulfonatoalkyl, -aryl, -alkaryl oder -aralkyl sowie für N-Alkyl-, N-Aryl, N-Alkaryloder N-Aralkylpyridin mit jeweils 1 bis 8 C-Atomen mit der Maßgabe steht, daß wenigstens einer der Reste R₁, R₂ oder R₃ ≠ Wasserstoff, Alkyl, Aryl, Aralkyl oder Alkaryl ist, wenigstens einer der Reste
R₄, R₅, R₆, R₇ und R₈ für eine Hydroxygruppe oder einen Polymerrest **steht,** der abgeleitet ist von Polyestern, Polymethylmethacrylaten, Polymethacrylsäuren, Polyacrylsäuren und deren Estern, Polymethacrylamiden, Polyacetal, Polyimiden und/oder Polyamiden sowie deren jeweiligen Monomerbausteinen und die verbliebenen Reste R4, R5, R6, R7 und R8 für Wasserstoff mit der Maßgabe steht, dass für den Fall, dass wenigstens einer der Reste R4, R5, R6, R7 oder R8 eine Hydroxygruppe ist, wenigstens einer der Reste R1, R2, und R3 für Wasserstoff, Alkyl, Aryl, Aralkyl oder Alkaryl, Alkyl-, Aryl-, Aralkyl- oder Alkarylsulfonat steht und
Me für ein Metallkation oder die Sättigung mit Wasserstoffatomen steht. Eine weitere Ausführungsform betrifft solche metallischen oder unmetallischen Porphyrinderivate der allgemeinen Formel I, wobei R₁, R₂ und R₃ jeweils für Wasserstoff, Alkyl, Aryl, Aralkyl oder Alkaryl, Alkyl-, Aryl-, Aralkyl- oder Alkarylsulfonat sowie für N-Alkyl-, N-Aryl, N-Alkaryl- oder N-Aralkylpyridin mit jeweils 1 bis 8 C-Atomen steht, mit der Maßgabe, dass wenigstens einer der Reste R₁, R₂ oder R₃ kein Wasserstoff, Alkyl, Aryl, Aralkyl oder Alkaryl ist, wenigstens einer der Reste R₄, R₅, R₆, R₇ und R₈ für einen Polymerrest steht, der abgeleitet von Polyestern, Polymethylmethacrylaten, Polymethacrylsäuren, Polyacrylsäuren und deren Estern, Polymethacrylamiden, Polyacetalen, Polyimiden und/oder Polyamiden sowie deren jeweiligen Monomerbausteine, wohingegen die anderen Wasserstoff sind; und Me für ein Metallkation oder die Sättigung mit Wasserstoff steht.
Me steht insbesondere für ein Metallkation mit halbbesetzter oder vollbesetzter d-Elektronenschale und Aluminium.

Überraschenderweise wurde gefunden, daß die erstmals zur Verfügung gestellten, gegebenenfalls hydrophilen Porphyrinderivate der allgemeinen Formel I effiziente Sensibilisatoren sind, die sich außerdem noch leicht kovalent an Polymere binden lassen oder gebunden sind. Durch die Modifikation der Substitutenten R₁, R₂ und R₃ mit polaren Gruppen läßt sich die Hydrophilie dieser Polymere in einem breiten Bereich steuern, so daß auf der einen Seite hydrophobe Polymere (d.h. keine polaren Gruppen) und auf der anderen Seite sogar wasserlösliche Polymere (hoher Anteil polarer Gruppen) herstellbar sind. Zwischen diesen beiden Extremen stehen hydrophile, aber wasserunlösliche Polymere. Außerdem kann die Wasserlöslichkeit durch den Polymerisationsgrad des Basispolymers gesteuert werden.

In einer besonders bevorzugten Ausführungsform der vorliegenden Erfindung sind die metallierten oder unmetallierten Porphyrinderivate der allgemeinen Formel I dadurch gekennzeichnet, daß R₄, R₅, R₇, und R₈ für Wasserstoff und R₆ für eine Hydroxygruppe steht. Diese Verbindungen dienen vorzugsweise als Ausgangsmaterial für die Umsetzung mit Monomerbausteinen oder Polymerbausteinen zur kovalenten Fixierung der Porphyrinderivate, insbesondere durch Umesterung vorhandener Estergruppen.

Ein weiterer Aspekt der Erfindung ist die Einführung von Sulfonsäuregruppen in die Alkyl-, Aryl-, Alaryl- oder Aralkylgruppen des Porphyrinderivats. Dabei kann der Sulfonierungsgrad über die Zugabe des Sulfonierungsmittel gezielt gesteuert werden, wodurch der hydrophile Charakter des Polymers geändert werden kann. Die Sulfonierung kann vor der Herstellung der Monomeren durchgeführt werden oder nach der Darstellung der Polymeren.

Ein weiterer Aspekt der Erfindung ist die Alkylierung der Pyridingruppen der Porphyrinderivate. Dabei kann der Alkylierungsgrad über die Zugabe des Alkylierungsmittels gezielt gesteuert werden. Die Alkylierung kann vor der Herstellung der Monomeren oder nach der Darstellung der Polymeren erfolgen. Für die Alkylierung sind Alkylderivate mit 1 bis 8 C-Atomen besonders geeignet.

Ein weiterer Aspekt der Erfindung ist die Einführung von Metallen insbesondere mit halbgefüllter d-Elektonenschale, nachdem das unmetallierte photosensitive Polymer vorliegt. Besonders bevorzugt enthalten die Porphyrinderivate Zink als zentrales Kation.

Ein besonderer Aspekt der Erfindung ist die Darstellung von porphyrinhaltigen Polymethylmethacrylaten über die Umesterung der vorhanden Estergruppen mit 5-(4-Hydroxyphenyl)-10,15,20-triarylporphyrin-derivaten. Dabei können die Porphyrine metalliert oder unmetalliert sein.

Eine weitere bevorzugte Ausführungsform der vorliegenden Erfindung besteht in einem Verfahren zur Herstellung von Porphyrinderivaten der allgemeinen Formel I durch Umsetzung von Porphyrinderivaten der allgemeinen Formel II 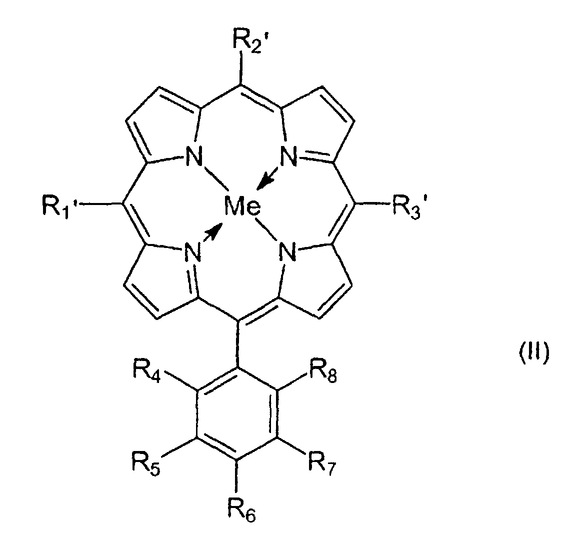 wobei
R_{1'}, R_{2'} und R_{3'} jeweils für Wasserstoff, Alkyl, Aryl, Alkaryl oder Aralkyl mit jeweils 1 bis 8 C-Atomen und der Maßgabe steht, daß wenigstens einer der Reste R_{1'},
R_{2'} oder R_{3'} ≠ Wasserstoff ist und
Me, R₄, R₅, R₆, R₇ und R₈ die obengenannte Bedeutung haben
mit einem Sulfonierungsmittel, gegebenenfalls gefolgt von anschließender Metallierung.

Besonders bevorzugt im Sinne der vorliegenden Erfindung wird als Sulfonierungsmittel Chlorsulfonsäure oder konzentrierte Schwefelsäure nach an sich bekannten Verfahren eingesetzt.

Ein weiteres Verfahren zur Herstellung von Porphyrinderivaten der allgemeinen Formel I besteht darin, daß man etwa vier Teile Pyrrol, etwa ein bis drei Pyridincarboxyaldehyd und etwa einen Teil Benzaldehyd mit nachfolgender N-Alkylierung sowie gegebenenfalls Metallierung umsetzt.

Eine weitere bevorzugte Ausführungsform des erfindungsgemäßen Verfahrens zur Herstellung von Porphyrinderivaten besteht darin, daß man die erhaltenen Porphyrine der allgemeinen Formel I, mit Polyestern, Polymethylmethacrylaten, Polymethacrylsäuren, Polyacrylsäuren und deren Estern, Polymethacrylamiden, Polyacetalen, Polyimiden und/oder Polyamiden oder deren jeweiligen Monomerbausteinen umsetzt.

Besonders bevorzugt im Sinne der vorliegenden Erfindung werden die obengenannten Porphyrinderivate mit Polymethylmethacrylat und/oder Polycarbonaten umgesetzt.

Ein weiterer Aspekt der Erfindung ist die Darstellung von Porphyrinderivaten über die Copolymerisation von Methacrylat mit metallierten und unmetallierten Monomeren der Ester aus Methacrylsäure oder Arcylsäure und 5-(4-Hydroxyphenyl)-10,15,20-arylporphyrin-Derivaten. Dabei können die letztgenannten Monomeren Sulfonsäuregruppen oder Pyridiniumsalze enthalten. Die so erhaltenen Photosensibilisatoren sind über eine kovalente Bindung an das Polymergerüst angebunden und ragen aus dem Polymer heraus. Sie sind keine im Inneren des Polymer eingebetteten Knotenpunkte.

Eine weitere Ausführungsform der vorliegenden Erfindung betrifft Photosensibilisätoren, die wenigstens ein Porphyrinderivat der allgemeinen Formel I in einer Menge von 0,1 bis 10 Gew.-% enthalten. Hierbei liegt das Porphyrinderivat der allgemeinen Formel I kovalent oder in nicht kovalent gebundener Form in einer Polymermatrix vor. Besonders bevorzugt im Sinne der vorliegenden Erfindung ist die kovalente Fixierung der Porphyrine in dem Trägermaterial.

Als polymere Trägermaterialien sind vor allem PMMA und Polycarbonate für technische Anwendungen interessant, da sie vorteilhafte Eigenschaften aufweisen: preiswert, mechanisch stabil, witterungsbeständig, lichtbeständig, transparent im Bereich der zur Anregung der Sensibilisatoren erforderlichen Lichtes.

Die Polymermatrix kann erfindungsgemäß bevorzugt aus Fasern, Granulaten, Vliesen, Geweben, Gewirken, Gestricken oder auch beliebigen Formkörpern, beispielsweise Membranen bestehen.

Ein weiterer Aspekt der Erfindung ist die Verwendung der photosensitiven Monomeren und Polymeren als Sensibilisatoren für photochemische Reaktionen. Die dargestellten Monomere und Polymere eignen sich für die Übertragung von aus Licht aufgenommener Energie auf chemische Substanzen.

Im Stand der Technik (WO 93/00815) wurden Versuche unternommen, Porphyrine an Polymere zu fixieren. Hierbei ging man so vor, daß regenerierte Cellulose mit TRPyP gefärbt wurde, wobei Cytotoxizität beobachtet wurde.

Für Anwendungen, bei denen jedoch eine Langzeitstabilität der polymerfixierten Sensibilisatoren in wäßriger Umgebung gefordert wird, ist es von Nachteil, die Porphyrinderivate an Cellulose zu binden, da Cellulose langsam hydrolysiert. Dieser Nachteil besteht nicht in dem Fall der kovalenten Bindung an PMMA. Zwar wurde im Stand der Technik gefunden, daß ein Zellwachstum bei einem direkten Kontakt mit Bakterienkulturen inhibiert wird. Die eingesetzten Porphyrine zeichnen sich aber dadurch aus, daß sie vierfach symmetrisch substituiert sind, kein Zentralatom beinhalten und positiv geladene 4-Alkylpyridine besitzen.

Bei eigenen Versuchen im Rahmen der vorliegenden Erfindung zeigte sich jedoch überraschenderweise, daß bei der Bestrahlung von an PMMA fixiertem 5-(4-Hydroxyphenyl)-10,15,20-triphenylporphyrin, das in direkten Kontakt zu einer luftgesättigten wäßrigen Phase keine Bildung von Singulett-Sauerstoff beobachtet werden konnte. Der Unterschied zum Stand der Technik, bei dem auch dieser Farbstoff verwendet wurde, ist möglicherweise dadurch erklärbar, daß ein direkter Kontakt zu der betreffenden Bakterienkultur gewährleistet war, während bei Versuchen gemäß der Erfindung eine Diffusionsbarriere an der Grenzschicht Polymer - Wasser zu überwinden war.

Ein wichtiger Unterschied der vorliegenden Erfindung zu TRPyP-Derivaten des Standes der Technik ist, daß sie symmetrisch gebaut sind und über keine funktionelle Gruppe zur kovalenten Bindung verfügen. Erfindungsgemäß wurden unsymmetrische Porphyrine synthetisiert, die insbesondere über eine Esterfunktion an ein Trägermaterial fixiert sind.

Der Einsatz des bekannten vierfach acryliertem Tetra-5,10,15,20-(4-hydroxyphenyl)prophyrin als ein Comonomer bei der Darstellung von photosensitivem PMMA schließt den Farbstoff als Vernetzungsstelle in die Polymerstruktur ein. Dadurch werden nur geringe Farbstoffeinheiten an die Oberfläche des Polymers gelangen und somit photosensitiv sein. Die Ausbeute an Singulett-Sauerstoff wird bezogen auf die Farbstoffanzahl erheblich reduziert. Bei den erfindungsgemäßen Porphyrinderivaten sind die Farbstoffeinheiten frei zugänglich, da sie nur über eine funktionelle Gruppe an das Trägermaterial gebunden sind.

Die Verwendung des bekannten, an PMMA fixierten 5-(4-Hydroxyphenyl)-10,15,20-triphenylporphyrin in wäßrigen Systemen ruft nach Versuchen der vorliegenden Erfindung keine Singulett-Sauerstoff-Bildung hervor. Die erfindungsgemäß hergestellten Farbstoffe besitzen hingegen eine höhere Hydrophilie und sind somit unter anderem in der Lage, gute Singulett-Sauerstoff-Bildungsraten zu erzeugen. Das an sich bekannte Verfahren zur Herstellung des Polymers über die radikalische Polymerisation benötigt 5 bis 6 Tage oder bei der Copolymerisation mit Styrol 2 Tage. Das erfindungsgemäß bevorzugte Verfahren über die Veresterung liefert ein Produkt innerhalb weniger Stunden.

Durch Modifikation der gebundenen Porphyrine gelang erfindungsgemäß nun trotzdem die Aktivierung von Sauerstoff. Die Modifikation besteht darin, negativ oder positiv geladene Gruppen an das Porphyringerüst anzubringen. Mit dieser Strategie wurde erstmals Porphyrinderivate der allgemeinen Formel I hergestellt, die als gemeinsame Merkmale enthalten:
- Ladung
- Verknüpfung

Eine weitere Aufgabe der vorliegenden Erfindung besteht in einer Verbesserung der Behandlung von insbesondere Wasser oder Wasser enthaltenden Umgebungen durch Bestrahlung mit aktinischem, d. h. sichtbarem Licht. Weiterhin besteht die Aufgabe der vorliegenden Erfindung in der Bereitstellung von Gegenständen oder Behältern zur Desinfektion und/oder Detoxifikation von Gegenständen in Sauerstoff und insbesondere Wasser und/oder Wasser enthaltenen Umgebungen.

Die vorgenannte Aufgabe wird in einer Ausführungsform gelöst durch die Verwendung der obengenannten Porphyrine zur Detoxifizierung und/oder Desinfektion von Sauerstoff und insbesondere Wasser oder Wasser enthaltenen Umgebungen und durch Bestrahlung mit aktinischem Licht.

Die besonderen Vorteile der vorliegenden Erfindung bestehen in einem geringen Energieaufwand zum Anlagenbetrieb. Es kann in einem Arbeitsschritt desinfiziert und dekontaminiert werden. Durch die Bindung an ein polymeres Trägermaterial ist eine Rückgewinnungsproblematik bei der vorliegenden Erfindung nicht gegeben. Die polymergebundenen Porphyrine verbleiben in dem System, so daß keine permanenten Kosten durch ständigen Zusatz von Verbindungen erforderlich sind. Es werden keine toxischen Nebenprodukte oder Farbstoffe gebildet, - auch treten keine Abbauprodukte der metallierten und/oder unmetallierten polymergebundenen Porphyrine auf. Da die Substanz nicht toxisch ist, sind keine zusätzlichen Sicherheitsmaßnahmen erforderlich. Das erfindungsgemäße Verfahren der Verwendung von metallierten und/oder unmetallierten polymergebundenen Porphyrinen ist somit auch für die Reinigung großer Volumina geeignet. In sonnenreichen Gegenden sind keine zusätzlichen Lichtquellen notwendig. In Kombination mit kleinen mobilen Einheiten kann das erfindungsgemäße Prinzip auch in entlegenen Gegenden eingesetzt werden.

Es war jedoch überraschend, daß die polymergebundenen Porphyrine bei Kontakt mit Wasser oder Wasser enthaltenden Umgebungen quellen und somit auch hier in vorteilhafter Weise Singulett-Sauerstoff bei der Bestrahlung mit aktinischem (sichtbarem) Licht bilden.

Besonders bevorzugt im Sinne der vorliegenden Erfindung werden die Porphyrine zur Behandlung von Wasser oder Wasser enthaltenen Umgebungen in Kläranlagen eingesetzt. Zur Nachreinigung von kontaminiertem Wasser kann dieses derart aufbereitet werden, daß das Wasser als Brauchwasser innerhalb der Anlage rückgeführt werden kann. Diese grundsätzliche Rückführungsmöglichkeit besteht nicht nur bei kommunalen und industriellen Kläranlagen sondern auch beispielsweise für die Lebensmittelindustrie, wo Obst und Gemüse zur Konservenherstellung gewaschen werden. In der Textilindustrie, insbesondere in Gerbereien und überall dort, wo große Mengen Wasser anfallen, welches man auf diesem Wege dem hausintemen Brauchwasserkreislauf wieder rückführen und damit die Kosten für Wasser beziehungsweise Abwasser erheblich reduzieren kann, ist mit Hilfe der vorliegenden Erfindung eine Behandlung des Wassers möglich. Die vorliegende Erfindung eignet sich auch insbesondere für die Behandlung von Wasch-/Bade- und Duschwasser von großen Hotelanlagen oder Campingplätzen. Auch ist mit Hilfe der vorliegenden Erfindung eine permanente Desinfektion von Schwimmbädern möglich, wobei der Chlorverbrauch und somit die Umwelt und Gesundheitsbelastung sowie die Betriebskosten erheblich gesenkt werden können.

Im Weltraum ist beispielsweise die Aufbereitung des Brauchwassers in transparenten Tanks möglich.

Darüber hinaus ist das erfindungsgemäße Verfahren einsetzbar zur Abluftreinigung oder Umluftreinigung, insbesondere von Luft aus Viehställen oder zum Desodorieren von Abluft- und Umluftströmen in Gebäuden, beispielsweise Kaufhäusern.

In einer besonders bevorzugten Ausführungsform der vorliegenden Erfindung werden die oben genannten Porphyrine in Form von Flachbett-Reaktoren oder Blasensäulen-Reaktoren eingesetzt.

Eine weitere bevorzugte Ausführungsform der vorliegenden Erfindung besteht darin, daß man die oben genannten Porphyrine als ortsfeste Strukturen, insbesondere Platten aus Vollmaterial oder beschichtete Platten sowie als Granulat, Pulver, Membran oder Filter einsetzt.

Neben den oben genannten wäßrigen Systemen, die mit Hilfe der vorliegenden Erfindung detoxifiziert oder dekontaminiert werden können, ist es in einer weiteren Ausführungsform der vorliegenden Erfindung möglich, Gegenstände aller Art mit den oben genannten Porphyrinen zu desinfizieren und/oder zu detoxifizieren. Besonders bevorzugt im Sinne der vorliegenden Erfindung werden daher diese Gegenstände in eine wäßrige oder Wasser enthaltende Umgebung eingebracht und so mit den Porphyrinen in Kontakt gebracht.

Eine besondere Ausführungsform der vorliegenden Erfindung besteht somit darin, Behälter oder Gegenstände zur Detoxifizierung und/oder Desinfektion von Wasser oder Wasser enthaltenden Umgebungen bereitzustellen. Im Sinne der vorliegenden Erfindung umfassen die genannten Behälter oder Gegenstände Oberflächenbereiche aus polymergebunden Porphyrinen. Hierzu werden an sich bekannte Behälter oder Gegenstände hergestellt, die wenigstens in Teilbereichen der Oberfläche die polymergebundenen Porphyrine enthalten.

So können die oben beschriebenen Behälter beispielsweise Reinigungsgeräte für Kontaktlinsen, Zahnersatz oder Zahnregulatorien umfassen. Eine weitere Ausführungsform der vorliegenden Erfindung besteht somit aus Gegenständen, die antibakterielle Beschichtungen von Oberflächen in der Medizintechnik umfassen, wobei diese Oberflächen metallierte oder unmetallierte polymergebundene Porphyrinderivate enthalten.

Die erfindungsgemäße Verwendung der Porphyrine für die Reinigung von Wasser oder Luft beruht auf der Produktion von Singulett-Sauerstoff. Der Einsatz dieses Agens wird in mehreren Publikationen beschrieben. So zum Beispiel in Archer et al. loc. cit.; in Banks, J. G., Board, R. G., Carter, J. and Dodge, A. D. , (1984) The cytotoxic and photodynamic inactivation of mico-organisms by Rose Bengal, J. Appl. Bacteriol., 58, 391-400; Dahl, T.A., Midden, W.R., and Hartman, P.E., (1987) Pure singlet oxygen cytotoxitiy for Bacteria, Photochem. Photobiol. 46, 345 - 352.

Eine allgemeine Übersicht der Reaktionen, die mit Singulett-Sauerstoff durchgeführt werden können, ist in Houben Weyl, Methoden der organischen Chemie, Photochemie Band II, Georg Thieme Verlag Stuttgart, 1975, S. 1465 beschrieben. Als chemische Substanzklassen werden dort Alkene, Aromaten, Heteroaromaten, Stickstoff-, Schwefel-, Phosphorverbindungen und andere aufgeführt. Diese können in ein höher oxidiertes Derivat überführt werden, die so für eine biologische Klärung des Wassers vorbereitet werden. Die höher oxidierten Stoffe im Falle der Schwefelverbindungen besitzen einen reduzierten Eigenduft, so daß hier eine desodorierende Wirkung zu beobachten ist.

Daraus läßt sich prinzipiell ableiten, daß Singulett-Sauerstoff produzierender Farbstoff grundsätzlich in Wasser als Sensibilisator für Wasserreinigung eingesetzt werden kann.

Die Reaktivität von Singulett-Sauerstoff in der Gasphase ist in verschiedenen Pulbikationen nachgewiesen worden, wie zum Beispiel in Eisenberg, Walter C.; DeSilva, Mutha, Journal, TELEAY, Tetrahedron Lett., EN, 31,41, 1990, 5857-5860, "Atmospheric gas phase generation of singlet oxygen by homogeneous photosensitization"; Borisov, A.V., Tsivenko, V.I., Myasinkov, I.A., Venediktor E.A., Journal RJPACAR, Russ. J. Phys. Chem. (Engl. Transl.) EN, 64,6,1990, 922 - 924, "Photostimulated formation and emission of singlet oxygen from the surface of metallo-tetraphenylporphins adsorbed on silica" und Borisov, A.V.; Tsivenko, V.I., Myasnikov, I.A. Journal, RJPCAR, Russ. J. Phys. Chem. (Engl. Transl.), EN, 66, 2, 1992, 307 - 308, "Photostimulated emission of singlet oxygen molecules into the gas phase from tetra(m-butoxyphenyl)porphin adsorbed on SiO₂".

Zur Behandlung von Abluft und Abgasen sind folgende Verfahren bekannt:
1. Abscheidung gasförmiger Stoffe durch Absorption, Kondensation, Membran-Permeation und Trockensorption
   Der Nachteil besteht in dem Verbrauch von chemischen Hilfsreagenzien, zum Beispiel Aktiv-Kohle, Kalk etc.
2. Abbau von Dioxinen und Furanen in Abgasen mit Wasserstoffperoxid
   Der Nachteil besteht in dem Verbrauch an Wasserstoffperoxid
3. Abscheidung gasförmiger Schadstoffe durch katalytische Reaktionen wie Oxydation, Reduktion, und Zersetzungsreaktionen. Edelmetallkatalysatoren, Schwermetallkatalysatoren und oxidische Katalysatoren kommen zum Einsatz.
   Der Nachteil besteht in dem Abrieb, wobei eine Vergiftung der Katalysatoren möglich ist. Teilweise sind die Katalysatoren nur bei erhöhten Temperaturen wirksam.
   Bei den Oxidationskatalysatoren wird meist teureres Platinmetall verwendet. Teures Ozon wird für die Entkeimung von Abluft eingesetzt. Der Überschuß muß wieder zerstört werden.
4. Abscheidung gasförmiger Schadstoffe durch biologische Reaktionen: Biowäscher (Absorption in einer Flüssigkeit, die mit Mikroorganismen versetzt ist), Biofilter (sowohl Adsorption an einer Feststoffoberfläche als auch Absorption in einer Flüssigkeit) und Biomembranfilterverfahren.

Die Verfahren sind dem Handbuch des Umweltschutzes und der Umweltschutztechnik, Band 3: Additiver Umweltschutz: Behandlung von Abluft und Abgasen, von H. Bauer (Hrsg.), Springer Verlag, Berlin 1996 entnommen.

Alle Verfahren, die zum Beispiel zur Herstellung von Gegenständen aus PMMA angewendet werden, können auch erfindungsgemäß verwendet werden, sofern die Temperaturen unter 200 °C liegen.

Eine weitere Ausführungsform der Erfindung umfaßt daher die Verwendung von Photosensibilisatoren gemäß der vorliegenden Erfindung zur Bildung von Singulett-Sauerstoff aus Triplett-Sauerstoff durch Einwirkung von sichtbarem Licht, insbesondere zur Reinigung von kontaminierten Flüssigkeiten, beispielsweise Wasser.

Eine besondere Ausführungsform der Erfindung besteht darin, daß Metallierung, Sulfonierung, Alkylierung erst am fertigen modifizierten Polymer erfolgen kann. Hierdurch ergibt sich insbesondere die Möglichkeit zur gezielteren Steuerung der jeweiligen Grade.

### Ausführungsbeispiele:

### Beispiel 1

### 5-(4-Hydroxyphenyl)-10,15,20-tris(4-sulfonatophenyl)porphyrin

In 10 ml konzentrierter Schwefelsäure wurden 500 mg 5-(4-Hydroxyphenyl)-10,15,20-triphenylporpyhrin gelöst. Die Lösung wurde 10 h bei einer Temperatur von 110 °C gerührt. Nach dem Abkühlen und der Neutralisation wurde das ausgefallene Produkt separiert. Eine Reinigung erfolgte über Säulenchromatographie an Silicagel mit Aceton/Petrolether als Elutionsmittel.

### Beispiel 2

### Zink(II)-5-(4-hydroxyphenyl)-10,15,20-tris(4-sulfonatophenyl)porphyrin

Eine Mischung aus 1g 5'-(4-Hydroxyphenyl)-10,15,20-tris(4-sulfonatophenyl)porphyrin und 54,5 mg Zinkacetat wurde in 250 ml Essigsäure und 50 ml Chloroform gelöst und zum Rückfluß erhitzt. Das nach dem Abkühlen ausgefallene Produkt wurde über Säulenchromatographie an Silicagel mit Aceton/Petrolether als Elutionsmittel gereinigt.

Eine Mischung aus 1 g Zink(II)- 5-(4-hydroxyphenyl)-10,15,20-triphenylporphyrin und Chloroform wurden bei 0 °C mit 60 µl Chlorsulfonsäure gelöst in 10 ml Chloroform versetzt. Nach 12 h wurde die Lösung auf Eis gegossen und die organische Phase separiert. Nach dem Abziehen des Lösungsmittels erhielt man das Produkt.

### Beispiel 3

### 5-(4-Hydroxyphenyl)-10,15,20-tris(N-methyl-4-pyridinium)porphyrin

Eine Mischung aus vier Teilen Pyrrol, drei Teilen Pyridin-4-carboxyaldehyd und einem Teil Benzaldehyd in Propionsäure wurde 1 h lang erhitzt. Das Lösungsmittel wurde abgezogen und der entstandene Rückstand mit DMF und Ether gewaschen. Zur weiteren Reinigung wurde das entstandene Produkt mit Hilfe der Säulenchromatographie an Silicagel mit Aceton/Petrolether als Elutionsmittet aufbereitet. Die Methylierung erfolgte mit Methyl-p-toluolsulfonat.

### Beispiel 4

### Zink(II)-5-(4-hydroxyphenyl)-10,15,20-tris(N-methyl-4-pyridinium)porphyrin

Eine Mischung aus 1 g 5-(4-Hydroxyphenyl)-10,15,20-tris(N-methyl-4-pyridinium)porphyrin und 54,5 mg Zinkacetat wurden in 250 ml Essigsäure und 50 ml Chloroform gelöst und zum Rückfluß erhitzt. Das nach dem Abkühlen ausgefallene Produkt wurde über Säulenchromatographie an Silicagel mit Aceton/Petrolether als Elutionsmittel gereinigt.

Eine Mischung aus 1 g Zink(II)-5-(4-hydroxyphenyl)-10,15,20-tris(pyridinium)porphyrin wurde mit 10 g Methyl-p-toluolsulfonat in DMF unter Erwärmung umgesetzt. Nach Abziehen des Lösungsmittels konnte das resultierende Produkt aus Aceton umkristallisiert werden.

### Beispiel 5

### 5-(4-Methacryloyloxyphenyl)-10,15,20-tripyridiniumporphyrin

In 20 ml DMF wurde 1 g 5-(4-Hydroxyphenyl)-10,15,20-tris(pyridinium)porphyrin gelöst und mit 128 µl Methacrylsäure in Gegenwart von 200 µl Triethylamin versetzt. Nach dem Abreagieren wurde das Lösungsmittel entfernt, das photosensitve Monomer mit Ether aufgenommen und filtriert. Nach dem Abziehen des Ethers wurde das reine Monomer gewonnen.

### Beispiel 6

### Zink(II)-5-(4-hydroxyphenyl)-10,15,20-tris(4-sulfonatophenyl)porphyrin (fixiert)

a) Eine Mischung aus 200 mg Polymethylmethacrylat (PMMA) und 280 mg Zink(II)-5-(4-hydroxyphenyl)-10,15,20-tris(4-sulfonatophenyl)porphyrin in Toluol wurde in Gegenwart von p.Toluolsulfonsäure erwärmt. Dabei wurde das Polymer umgeestert. Durch mehrfaches Abdestillieren des Toluols wurde der Farbstoff vollständig fixiert.
b) 74 mg Zink(II)-5-(4-methacryloyloxyphenyl)-10,15,20-tris(4-sulfonatophenyl)porphyrin wurden mit 10 ml Methacrylsäuremethylester in DMF gelöst. Die Polymerisation wurde mit Azo-bis-isobutyronitril (AIBN) gestartet. Das Entfernen des Lösungsmittels lieferte das gewünschte Polymer als rotes Pulver.
c) 10 g des an PMMA fixierten Zink(II)-5-(4-hydroxyphenyl)-10,15,20-triphenylporphyrins wurde in Chloroform gelöst und mit 6 ml Chlorsulfonsäure vesetzt. Nach dem Neutralisieren und Abziehen des Lösungsmittels wurde das gewünschte Polymer gewonnen.
d) 10 g des an PMMA fixierten 5-(4-Hydroxyphenyl)-10,15,20-tris(4-sulfonatophenyl)porphyrins wurden in 200 ml Chloroform gelöst und mit Zinkacetat versetzt. Nach dem Erwärmen und Abziehen des Lösungsmittels wurde das gesuchte Polymer isoliert.

### Beispiel 7

### Singulett-Sauerstoff-Produktion der Farbstoffe (Porphyrinderivate)

Die Befähigung der Farbstoffe Singulett-Sauerstoff zu produzieren wurde über einen einfachen Test geprüft. Eine wäßrige Lösung mit einem Überschuß an Imidazol (0,01 mol/l) und einer Konzentration von 3 x 10⁻⁵ mol/l p-Nitrosodimethylanilin (RNO) wurde unter ständigem Rühren während der Messung mit Luft beziehungsweise Sauerstoff gesättigt. Imidazol wirkte als Akzeptor für Singulett-Sauerstoff, und das intermediär gebildete Endoperoxid induzierte eine Bleichung des RNO, die bei einer Wellenlänge von 440 nm spektrophotometrisch verfolgt wurde.

10 ml dieser RNO-Lösung wurden mit Zink(II)-5-(4-hydroxyphenyl)-10,15,20-tris(4-sulfonatophenyl)porphyrin (2ppm) versetzt und bestrahlt. Die Singulett-Sauerstoff-Bildungsrate wurde mit 8,8 x 10⁻⁸ mol/l/s bestimmt. Zum Vergleich besaß Rose Bengal einen Wert von 2,6 x 10⁻⁸ mol/l/s.

Das Polymer von Beispiel 6c) wurde in Suspension dem genannten Test unterworfen und bestrahlt. Nach Normierung wurde ein Wert von 3,0 x 10⁻⁸ mol/l/s ermittelt.

### Beispiel 8

### Versuch zur Bestimmung von Singulett-Sauerstoff Produktionsraten verschiedener Sensibilisatoren in Wasser

In Gegenwart von Imidazol oder Fufurylalkohol als Akzeptor wurde bei Bestrahlung mit polychromatischem Licht p-Nitrosodimethylanilin (RNO) gebleicht Diese Reaktion wurde von F.Le Guern, C. Bied-Charreton, J. Faure, Bull. Soc. Chem. Fr. 130, 753 (1993) verwendet, um Produktionsraten von Singulett-Sauerstoff von Sensibilisatoren zu bestimmen.

Der Aufbau der verwendeten Bestrahlungsapparatur war wie folgt:

In einer Petrischale wurde eine Lösung mit einem Überschuß an Imidazol und bekannter Konzentration an Nitrosodimethylanilin (RNO) eingefüllt. Die Lösung wurde permanent mit Sauerstoff gesättigt. Durch Zugabe des zu untersuchenden Farbstoffs und Bestrahlung mit einer konstanten Lichtquelle konnte die Bleichung des RNO spektroskopisch verfolgt werden. Die Konzentrationsabnahme des RNO wurde gegen die Zeit aufgetragen. Die Steigerung der resultierenden Geraden war ein Maß für die Produktionsrate an Singulett-Sauerstoff.

Zum Vergleich werden die Ergebnisse mit dem bekannten Sensibilisator Rose Bengal gegenübergestellt.

Für verschiedene Sensibilisatoren wurden so folgende Singulett-Sauerstoff Produktionsraten ermittelt:

| Farbstoff | Produktionsrate an Singulett-Sauerstoff Δc(¹O₂)/Δt[(mol/l)/s] |
|---|---|
| Rose Bengal* | 1,2 10⁻⁸ |
| Zink(II)5-(4-hydroxyphenyl)-10,15,20-tri(4-sulfonatophenyl)porphyrin an Polymethylmethacrylat gebunden** | 8,8 10⁻⁸ |
| Zink(II)5-(4-hydroxyphenyl)-10,15,20-tri(4-methyl-pyridino)porphyrin an Polymethylmethacrylat gebunden*** | 1,8 10⁻⁸ |

| | |
|---|---|
| * Vergleich, homogen gelöst | |
| ** erhältlich nach Beispiel 6 | |
| *** erhältlich nach Beispiel 4 | |

### Beispiel 9

### Nachweis des Abbaus von chemischen Substanzen mit Hilfe eines Porphyrin-Polymers

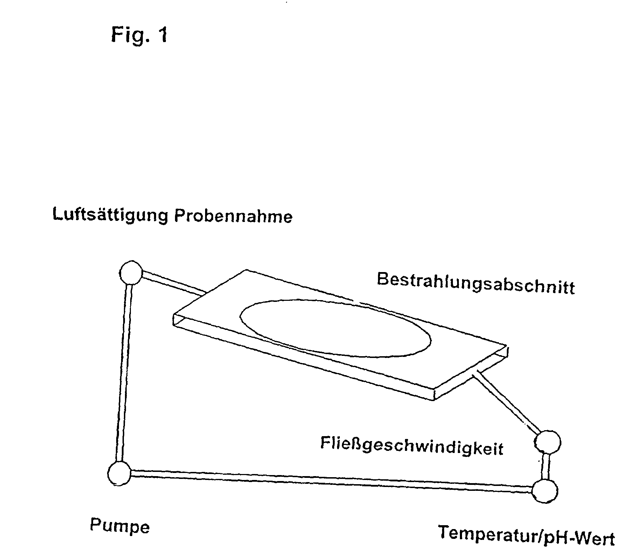

Für den Abbau von chemischen Substanzen wurde ein Fallfilmreaktor hergestellt. In ihm können sowohl homogen gelöste Sensibilisatoren wie auch PMMA-Platten, die aus photosensitivem Material angefertigt sind, untersucht werden. Die Fig. 1 zeigt den schematischen Aufbau.

Als Testsubstanz wurde das Pestizid Phosphorsäuretributylthioester (DEF) eingesetzt, das in einigen Ländern als verunreinigende Substanz im Grundwasser vorkommt. Der Nachweis der Substanz wurde mittels GC geführt unter Verwendung von Aufkonzentrierungs-Techniken.

Es wurden folgende Ergebnisse ermittelt, wobei Cᵢ die Ausgangskonzentration und und C, die Endkonzentration darstellen.

| Farbstoff | Cᵢ (DEF) [ppm] | C_{f} (DEF) [ppm] |
|---|---|---|
| Rose Bengal* | 3,2 | 0,23 |
| Zink(II)5-(4-hydroxyphenyl)-10,15,20-tri(4-methyl-pyridino)porphyrin an Polymethylmethacrylat gebunden*** | 3,04 | 0,77 |

| | | |
|---|---|---|
| * Vergleich, homogen gelöst | | |
| *** erhältlich nach Beispiel 4 | | |

### Beispiel 10

### Nachweis der Desinfektion mit Hilfe eines Porphyrin-Polymers

Für die Desinfektionsversuche wurde folgende Apparatur verwendet.

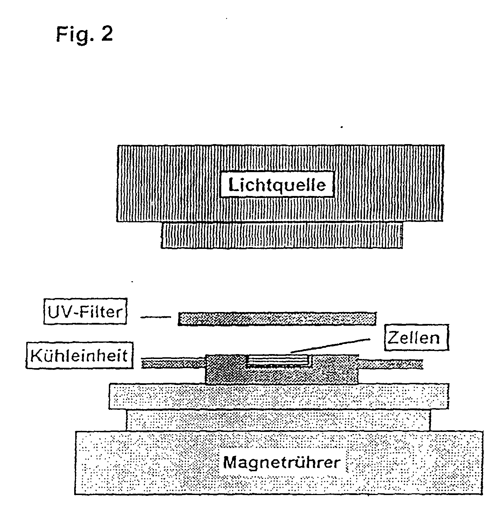

Als Lichtquelle wurde eine 1000 W Xenon-Lampe der Firma Polytec benutzt. Um den UV-Anteil der Lampe herauszufiltem, wurden die Proben mit 3 mm dickem Pyrexglas abgedeckt. Das Spektrum der Lampe unter dem Pyrexglas wurde überprüft und stimmte weitestgehend mit dem der Sonne überein. Um die Temperatur während des Versuchs kontrollieren bzw. konstant halten zu können, wurden die Proben in einer Kühleinheit bestrahlt und während der Bestrahlung mit einem Magnetrührer durchmischt. Den schematischen Aufbau der Bestrahlungseinheit zeigt die Fig. 2.

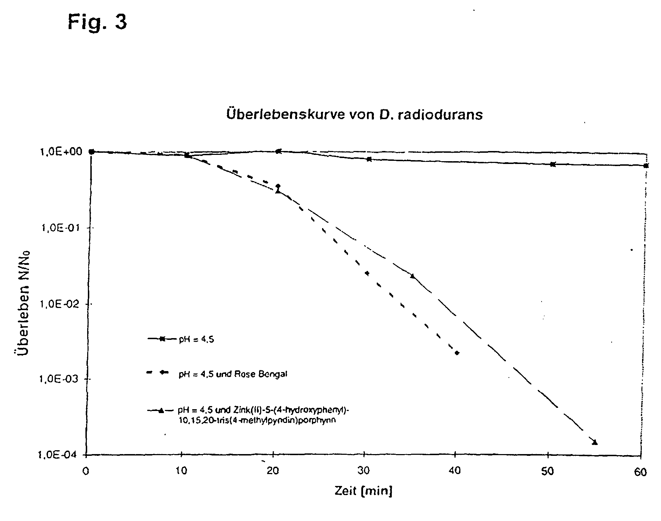

Für die Versuche mit Farbstoffen wurden die Zellen von D. radiodurans als Versuchsobjekt ausgewählt. Die Versuche lieferten die Überlebenskurven, die in Fig. 3 dargestellt sind.

### Beispiel 11

### Versuche zur Bestimmung von Singulett-Sauerstoff Produktionsraten verschiedener Sensibilisatoren in einem nicht wäßrigen Medium

Es wurde das gleiche Nachweissystem verwendet, wie in Beispiel 8 beschrieben, nur wurde das Lösungsmittel Wasser durch wasserfreies Ethylacetat ersetzt. Es wurden folgende Ergebnisse ermittelt.

| Farbstoff | Produktionsrate an Singulett-Sauerstoff Δc(¹O₂)/Δt [(mol/l)/s] |
|---|---|
| Rose Bengal* | 1,7 10⁻⁸ |
| Zink(II)5-(4-hydroxyphenyl)-10,15,20-tri(4-methyl-pyridino)porphyrin an Polymethylmethacrylat gebunden*** | 4,4 10⁻⁸ |

| | |
|---|---|
| * Vergleich, homogen gelöst | |
| *** erhältlich nach Beispiel 4 | |

## Patentansprüche

1. Metallierte oder unmetallierte Porphyrinderivate der allgemeinen Formel I 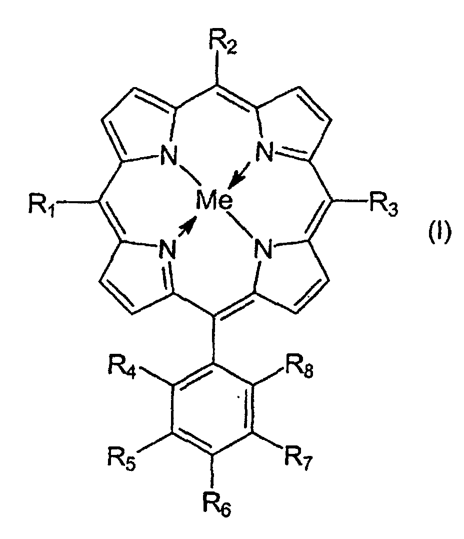 wobei
R₁, R₂ und R₃ jeweils für Wasserstoff, Alkyl, Aryl, Aralkyl oder Alkaryl, Sulfonatoalkyl, -aryl, -alkaryl oder -aralkyl sowie für N-Alkyl-, N-Aryl, N-Alkaryl oder N-Aralkylpyridin mit jeweils 1 bis 8 C-Atomen mit der Maßgabe steht, dass wenigstens einer der Reste R₁, R₂ oder R₃ ≠ Wasserstoff, Alkyl, Aryl, Aralkyl oder Alkaryl ist,
wenigstens einer der Reste R₄, R₅, R₆, R₇ und R₈ für eine Hydroxygruppe oder einen Polymerrest steht, der abgeleitet ist von Polyestern, Polymethylmethacrylaten, Polymethacrylsäuren, Polyacrylsäuren und deren Estern, Polymethacrylamiden, Polyacetalen, Polyimiden und/oder Polyamiden sowie deren jeweiligen Monomerbausteine und die verbliebenen Reste R₄, R₅, R₆, R₇ und R₈ für Wasserstorf mit der Maßgabe steht, dass für den Fall, dass wenigstens einer der Reste R₄, R₅, R₆, R₇ oder R₈ eine Hydroxygruppe ist, wenigstens einer der Reste R₁, R₂ und R₃ für Wasserstoff, Alkyl, Aryl, Aralkyl oder Alkaryl, Sulfonatoalkyl, -aryl, -alkaryl oder - aralkyl steht und
Me für ein Metallkation oder die Sättigung mit Wasserstoffatomen steht.

2. Metallierte oder unmetallierte Porphyrinderivate gemäß Anspruch 1, der allgemeinen Formel I, wobei R₁, R₂ und R₃ jeweils für Wasserstoff, Alkyl, Aryl, Aralkyl oder Alkaryl, Alkyl-, Aryl-, Aralkyl- oder Alkarylsulfonat sowie für N-Alkyl-, N-Aryl, N-Alkaryl- oder N-Aralkylpyridin mit jeweils 1 bis 8 C-Atomen steht, mit der Maßgabe, dass wenigstens einer der Reste R₁, R₂ oder R₃ kein Wasserstoff, Alkyl, Aryl, Aralkyl oder Alkaryl ist, wenigstens einer der Reste R₄, R₅, R₆, R₇ und R₈ für einen Polymerrest steht, der abgeleitet ist von Polyestern, Polymethylmethacrylaten, Polymethacrylsäuren, Polyacrylsäuren und deren Estern, Polymethacylamiden, Polyacetalen, Polyimiden und/oder Polyamiden sowie deren jeweiligen Monomerbausteine, wohingegen die anderen Wasserstoff sind; und Me für ein Metallkation oder die Sättigung mit Wasserstoff steht.

3. Metallierte oder unmetallierte Porphyrinderivate gemäß Anspruch 1 der allgemeinen Formel I, wobei
R₄, R₅, R₇ und R₈ für Wasserstoff und R₆ für eine Hydroxygruppe steht.

4. Metallierte oder unmetallierte Porphyrinderivate gemäß Anspruch 1 der allgemeinen Formel I, wobei Pyridin-N-Alkyl für Pyridin-N-Methyl steht.

5. Metallierte Porphyrine gemäß Anspruch 1 der allgemeinen Formel I, wobei Me für Zn steht.

6. Verfahren zur Herstellung von Porphyrinderivaten der allgemeinen Formel I gemäß Anspruch 1 durch Umsetzung von Porphyrinen der allgemeinen Formel II 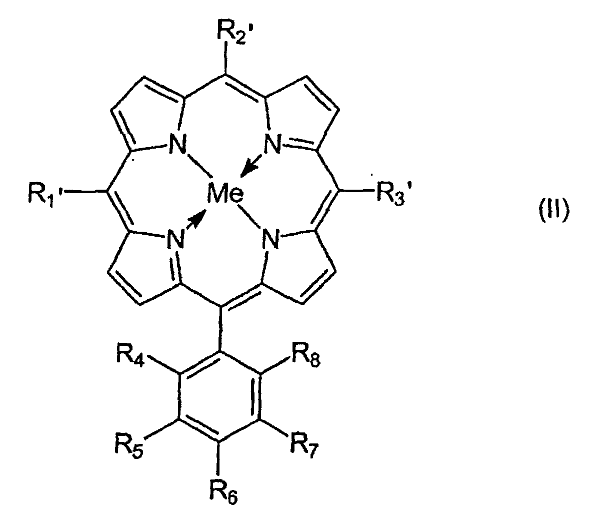 wobei
R_{1'}, R_{2'} und R_{3'} jeweils für Wasserstoff, Alkyl, Aryl, Alkaryl oder Aralkyl mit jeweils 1 bis 8 C-Atomen mit der Maßgabe steht, dass wenigstens einer der Reste R_{1'}, R_{2'} oder R_{3'} ≠ Wasserstoff ist und
Me, R₄, R₅, R₆, R₇ und R₈ die obengenannte Bedeutung haben, mit einem Sulfonierungsmittel, gegebenenfalls gefolgt von anschließender Metallierung.

7. Verfahren nach Anspruch 6, **dadurch gekennzeichnet, dass** man als Sulfonierungsmittel Chlorsulfonsäure oder konzentrierte Schwefelsäure einsetzt.

8. Verfahren zur Herstellung von Porphyrinderivaten der allgemeinen Formel I gemäß Anspruch 1 durch Umsetzung von vier Teilen Pyrrol, ein bis drei Teilen Pyridincarboxyaldehyd und einem Teil Benzaldehyd mit nachfolgender N-Alkylierung sowie gegebenenfalls Metallierung.

9. Verfahren nach Anspruch 6 oder 8, **dadurch gekennzeichnet, dass** man die erhaltenen Porphyrinderivate der allgemeinen Formel I mit Polyestern, Polymethylmethacrylaten, Polymethacrylsäuren, Polyacrylsäuren und deren Ester, Polymethacrylamiden, Polyacetalen, Polyimiden und/oder Polyamiden oder deren jeweiligen Monomerbausteinen umsetzt.

10. Verfahren nach Anspruch 9, **dadurch gekennzeichnet, dass** man Porphyrinderivate der allgemeinen Formel I mit Polymethylmethacrylat oder Polycarbonat umsetzt.

11. Photosensibilisator umfassend wenigstens ein Porphyrinderivat der allgemeinen Formel I gemäß Anspruch 2 in einer Menge von 0,1 bis 10 Gew.-%.

12. Photosensibilisator nach Anspruch 11, **dadurch gekennzeichnet, dass** das Porphyrin der allgemeinen Formel I kovalent gebunden mit einer Polymermatrix vorliegt.

13. Photosensibilisator nach Anspruch 12, **dadurch gekennzeichnet, dass** die Polymermatrix Fasern, Granulate, Pulver, Vliese, Gewebe, Gewirke, Gestricke oder Formkörper, insbesondere Membranen, umfasst.

14. Verwendung von Photosensibilisatoren nach einem oder mehreren der Ansprüche 11 bis 13 zur Auslösung photochemischer Reaktionen durch Einwirkung von sichtbarem Licht.

15. Verwendung von Photosensibilisatoren nach Anspruch 14 zur katalytischen Bildung von Singulett-Sauerstoff aus Triplett-Sauerstoff durch Einwirkung von sichtbarem Licht.

16. Verwendung von metallierten und/oder unmetallierten polymergebundenen Porphyrinen nach Anspruch 14 zur Detoxifizierung und/oder Desinfektion von Sauerstoff und insbesondere Wasser oder Wasser enthaltenden Umgebungen durch Bestrahlung mit aktinischem Licht.

17. Verwendung nach Anspruch 14 zur Aufbereitung von Brauchwasser, insbesondere in Wasserkreisläufen; als Zwischenreinigung oder Endreinigung in industriellen oder kommunalen Kläranlagen; zur Waschwasser-Reinigung in der Lebensmittelindustrie, insbesondere beim Waschen von Obst und Gemüse vor der Konservierung; zur Reinhaltung von Wasser-Speichern; zur Trinkwasserstabilisierung; in Kombination mit Gaswäschern, bei denen belastete Waschwässer anfallen; bei der Aufbereitung von Wasch-, Bade- und Duschwasser von industriellen und kommunalen Anlagen, insbesondere Hotelanlagen oder Campingplätzen; zur Reinigung von Schwimmbadwässern, insbesondere in Kombination mit solarer Wassererwärmung; zur Abluftreinigung oder Umluftreinigung, insbesondere von Luft aus Viehställen oder zum Desodorieren von Abluft- und Umluftströmen in Gebäuden.

18. Verwendung nach Anspruch 14, **dadurch gekennzeichnet, dass** man die Porphyrine als ortsfeste Strukturen, insbesondere Platten aus Vollmaterial oder beschichtete Platten sowie als Granulate, Pulver, Membranen oder Filter einsetzt.

19. Verwendung nach Anspruch 14, **dadurch gekennzeichnet, dass** man Porphyrine in Form von Flachbett-Reaktoren oder Blasensäulen-Reaktoren einsetzt.

20. Verwendung von Porphyrinderivaten der allgemeinen Formel I nach einem der Ansprüche 2 bis 4, **dadurch gekennzeichnet, dass** man die Porphyrine zur Desinfektion und/oder Detoxifikation von Gegenständen aller Art einsetzt.

21. Behälter oder Gegenstände zur Detoxifizierung und/oder Desinfektion von Gegenständen, Wasser oder Wasser enthaltenden Umgebungen, umfassend Oberflächenbereiche aus polymergebundenen Porphyrinen nach einem der Ansprüche 2 bis 4.

22. Behälter nach Anspruch 21, umfassend Reinigungsgeräte für Kontaktlinsen, Zahnersatz oder Zahnregulatorien.

23. Gegenstände nach Anspruch 21, umfassend antibakterielle Beschichtungen von Oberflächen in der Medizintechnik.

## Claims

1. Metallated or unmetallated porphyrin derivatives of general formula I 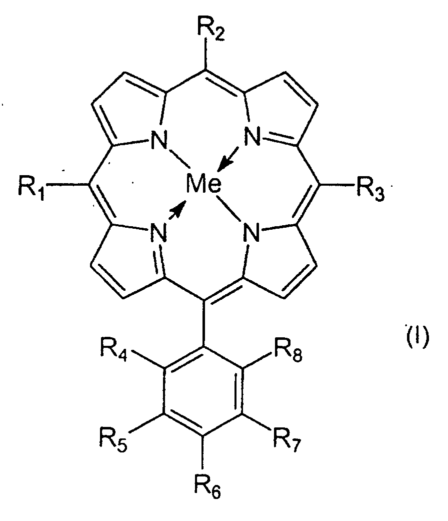 wherein
R_{1,} R₂ and R₃ respectively represent hydrogen, alkyl, aryl, aralkyl or alkaryl, sulfonatoalkyl, -aryl, -alkaryl or -aralkyl, or N-alkyl-, N-aryl-, N-alkaryl- or N-aralkylpyridine each having from 1 to 8 carbon atoms, with the proviso that at least one of R₁, R₂ or R₃ is not hydrogen, alkyl, aryl, aralkyl or alkaryl;
at least one of residues R₄, R₅, R₆, R₇ and R₈ represents a hydroxy group or a polymer residue derived from polyesters, polymethyl methacrylates, polymethacrylic acids, polyacrylic acids and their esters, polymethacrylamides, polyacetals, polyimides and/or polyamides, and their respective monomer units; and the remaining residues R₄, R₅, R₆, R₇ and R₈ represent hydrogen, with the proviso that, if at least one of residues R₄, R₅, R₆, R₇ and R₈ is a hydroxy group, then at least one of residues R₁, R₂ and R₃ represents hydrogen, alkyl, aryl, aralkyl or alkaryl, sulfonatoalkyl, -aryl, -alkaryl or -aralkyl; and
Me represents a metal cation or saturation with hydrogen atoms.

2. Metallated or unmetallated porphyrin derivatives according to claim 1 of general formula I wherein R₁, R₂ and R₃ respectively represent hydrogen, alkyl, aryl, aralkyl or alkaryl, sulfonatoalkyl, -aryl, -alkaryl or -aralkyl, or N-alkyl-, N-aryl-, N-alkaryl- or N-aralkylpyridine each having from 1 to 8 carbon atoms, with the proviso that at least one of R₁, R₂ or R₃ is not hydrogen, alkyl, aryl, aralkyl or alkaryl, at least one of residues R₄, R₅, R₆, R₇ and R₈ represents a polymer residue derived from polyesters, polymethyl methacrylates, polymethacrylic acids, polyacrylic acids and their esters, polymethacrylamides, polyacetals, polyimides and/or polyamides, and their respective monomer units, the remaining being hydrogens; and Me represents a metal cation or saturation with hydrogen atoms.

3. Metallated or unmetallated porphyrin derivatives according to claim 1 of general formula I wherein R₄, R₅, R₇ and R₈ represent hydrogen, and R₆ represents a hydroxy group.

4. Metallated or unmetallated porphyrin derivatives according to claim 1 of general formula I wherein pyridine-N-alkyl represents pyridine-N-methyl.

5. Metallated porphyrins according to claim 1 of general formula I wherein Me represents Zn.

6. A process for the preparation of porphyrin derivatives of general formula I according to claim 1 by reacting porphyrins of general formula II 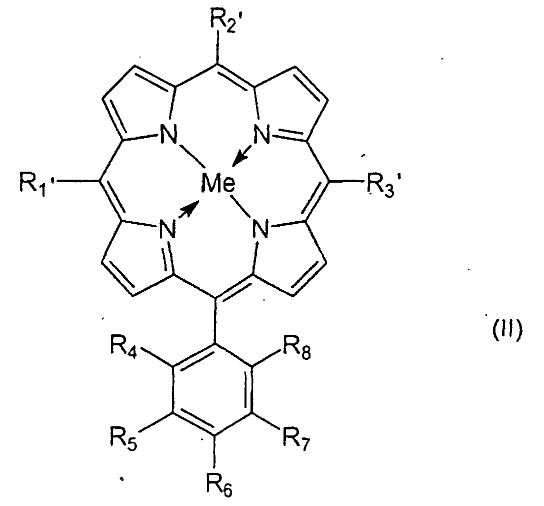 wherein
R_{1'}, R_{2'} and R_{3'} respectively represent hydrogen, alkyl, aryl, alkaryl or aralkyl having from 1 to 8 carbon atoms, with the proviso that at least one of R_{1'}, R_{2'} or R_{3'} is not hydrogen; and
Me, R₄, R₅, R₆, R₇ and R₈ are as defined above;
with a sulfonating agent, optionally followed by a subsequent metallation.

7. The process according to claim 6, **characterized in that** chlorosulfonic acid or concentrated sulfuric acid is employed as said sulfonating agent.

8. A process for the preparation of porphyrin derivatives of general formula I according to claim 1 by reacting four parts of pyrrole, one to three parts of pyridinecarboxyaldehyde and one part of benzaldehyde followed by N-alkylation and optionally metallation.

9. The process according to claim 6 or 8, **characterized by** reacting the obtained porphyrin derivatives of general formula I with polyesters, polymethyl methacrylates, polymethacrylic acids, polyacrylic acids and their esters, polymethacrylamides, polyacetals, polyimides and/or polyamides, or their respective monomer units.

10. The process according to claim 9, **characterized by** reacting porphyrin derivatives of general formula I with polymethyl methacrylate or polycarbonate.

11. A photosensitizer comprising at least one porphyrin derivative of general formula I according to claim 2 in an amount of from 0.1 to 10% by weight.

12. The photosensitizer according to claim 11, **characterized in that** said porphyrin of general formula I is present in a polymer matrix in a covalently bonded form.

13. The photosensitizer according to claim 12, **characterized in that** said polymer matrix comprises fibers, granules, powders, non-woven, woven or knitted fabrics or shaped bodies, especially membranes.

14. Use of photosensitizers according to one or more of claims 11 to 13 for initiating photochemical reactions by the action of visible light.

15. The use of photosensitizers according to claim 14 for the catalytic formation of singlet oxygen from triplet oxygen by the action of visible light.

16. The use of metallated and/or unmetallated porphyrins bound to polymers according to claim 14 for the detoxification and/or disinfection of oxygen and especially water or water-containing environments by irradiation with actinic light.

17. The use according to claim 14 for the processing of service water, especially in water cycles; as intermediary purification or final purification in industrial or communal waste water treatment plants; for the purification of washing water in the food industry, especially in the washing of fruits and vegetables prior to canning; for keeping water storage tanks clean; for drinking water stabilization; in combination with gas scrubbers in which loaded washings are obtained; in the treatment of washing, bathing and showering water of industrial and communal plants, especially hotels or camping sites; for the purification of swimming pool waters, especially in combination with solar water heating; for the purification of exhaust air or circulated air, especially of air from cattle sheds, or for deodorizing exhaust air or recirculated air streams in buildings.

18. The use according to claim 14, **characterized in that** said porphyrins are used as stationary structures, especially sheets of solid material or coated sheets, and as granules, powders, membranes or filters.

19. The use according to claim 14, **characterized in that** porphyrins are employed in the form of flat-bed reactors or bubble column reactors.

20. Use of porphyrin derivatives of general formula I according to any of claims 2 to 4, **characterized in that** said porphyrins are employed for disinfecting and/or detoxificating all kinds of objects.

21. Containers or articles for the detoxification and/or disinfection of objects, water or water-containing environments, comprising surface regions made of porphyrins bound to a polymer according to any of claims 2 to 4.

22. The containers according to claim 21, comprising purification devices for contact lenses, dental prostheses or dental regulatory devices.

23. The articles according to claim 21, comprising antibacterial coatings of surfaces in medical engineering.

## Revendications

1. Dérivés de porphyrine métallés ou non métallés de formule générale I : 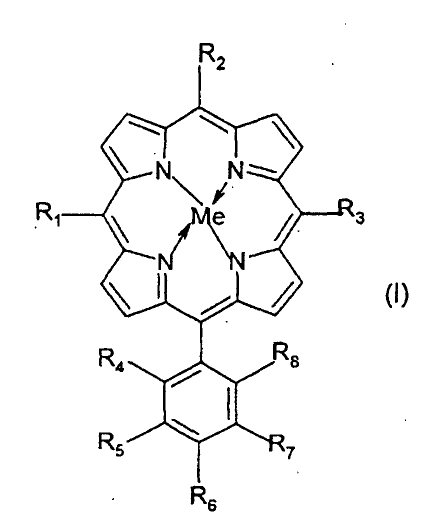 dans laquelle
R₁, R₂ et R₃ représentent respectivement hydrogène, alkyle, aryle, aralkyle ou alkaryle, sulfonatoalkyle, sulfonatoaryle, sulfonatoalkaryle ou sulfonatoaralkyle ainsi que N-alkylpyridine, N-arylpyridine, N-alkarylpyridine ou N-aralkylpyridine, ayant respectivement de 1 à 8 atomes de C, avec la condition qu'au moins l'un des résidus R₁, R₂ et R₃ soit différent de hydrogène, alkyle, aryle, aralkyle ou alkaryle,
au moins l'un des résidus R₄, R₅, R₆, R₇ et R₈ représente un groupe hydroxy ou un résidu polymère issu de polyesters, poly(méthacrylates de méthyle), poly(acides méthacryliques), poly(acides acryliques) et leurs esters, polyméthacrylamides, polyacétals, polyimides et/ou polyamides ainsi que leurs composants monomères respectifs et les résidus restants R₄, R₅, R₆, R₇ et R₈ représentent hydrogène, avec la condition que dans le cas où au moins l'un des résidus R₄, R₅, R₆, R₇ et R₈ représente un groupe hydroxy, au moins l'un des résidus R₁, R₂ et R₃ représente hydrogène, alkyle, aryle, aralkyle ou alkaryle, sulfonatoalkyle, sulfonatoaryle, sulfonatoalkaryle ou sulfonatoaralkyle et Me représente un cation métallique ou la saturation avec des atomes d'hydrogène.

2. Dérivés de porphyrine métallés ou non métallés selon la revendication 1, de formule générale I, dans laquelle R₁, R₂ et R₃ représentent respectivement hydrogène, alkyle, aryle, aralkyle ou alkaryle, alkylsulfonate, arylsulfonate, alkarylsulfonate ou aralkylsulfonate ainsi que N-alkylpyridine, N-arylpyridine, N-alkarylpyridine ou N-aralkylpyridine, ayant respectivement de 1 à 8 atomes de C avec la condition qu'au moins l'un des résidus R₁, R₂ et R₃ ne soit pas hydrogène, alkyle, aryle, aralkyle ou alkaryle, au moins l'un des résidus R₄, R₅, R₆, R₇ et R₈ représente un résidu polymère issu de polyesters, poly(méthacrylates de méthyle, poly(acides méthacryliques), poly(acides acryliques) et leurs esters, polyméthacrylamides, polyacétals, polyimides et/ou polyamides ainsi que leurs composants monomères respectifs, les autres résidus étant en revanche hydrogène ; et Me représente un cation métallique ou la saturation avec des atomes d'hydrogène.

3. Dérivés de porphyrine métallés ou non métallés selon la revendication 1, de formule générale I, dans laquelle R₄, R₅, R₇ et R₈ représentent hydrogène et R₆ représente un groupe hydroxy.

4. Dérivés de porphyrine métallés ou non métallés selon la revendication 1, de formule générale I, dans laquelle pyridine-N-alkyle représente la pyridine-N-méthyle.

5. Porphyrine métallée selon la revendication 1, de formule générale I, dans laquelle Me représente Zn.

6. Procédé de préparation de dérivés de porphyrine de formule générale I selon la revendication 1, par réaction des porphyrines de formule générale II 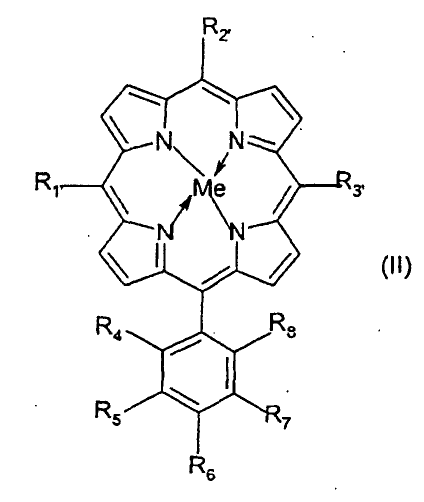 dans laquelle
R_{1'}, R_{2'} et R_{3'} représentent respectivement hydrogène, alkyle, aryle, aralkyle ou alkaryle, ayant respectivement de 1 à 8 atomes de C, avec la condition qu'au moins l'un des résidus R_{1'}, R_{2'} et R_{3'} soit différent de hydrogène, et Me, R₄, R₅, R₆, R₇ et R₈ aient les significations susmentionnées,
avec un agent de sulfonation, éventuellement suivie d'une métallation consécutive.

7. Procédé selon la revendication 6, **caractérisé en ce que** l'on met en oeuvre à titre d'agent de sulfonation de l'acide chlorosulfonique ou de l'acide sulfurique concentré.

8. Procédé de préparation de dérivés de porphyrine de formule générale I selon la revendication 1, par réaction de quatre parties de pyrrol, une à trois parties de pyridine carboxyaldéhyde et une partie de benzaldéhyde, suivie d'une N-alkylation et éventuellement d'une métallation.

9. Procédé selon la revendication 6 ou 8, **caractérisé en ce que** l'on fait réagir les dérivés de porphyrine obtenus de formule générale I avec des polyesters, poly(méthacrylates de méthyle), poly(acides méthacryligues), poly(acides acryliques) et leurs esters, polyméthacrylamides, polyacétals, polyimides et/ou polyamides ou leurs composants monomères respectifs.

10. Procédé selon la revendication 9, **caractérisé en ce que** l'on fait réagir des dérivés de porphyrine de formule générale I avec du poly(méthacrylate de méthyle) ou un polycarbonate.

11. Photosensibilisateur comprenant au moins un dérivé de porphyrine de formule générale I selon la revendication 2 en une quantité allant de 0,1 à 10 % en poids.

12. Photosensibilisateur selon la revendication 11, **caractérisé en ce que** la porphyrine de formule générale I est liée de manière covalente à une matrice polymère.

13. Photosensibilisateur selon la revendication 12, **caractérisé en ce que** la matrice polymère comprend des fibres, granulés, poudres, matelas de fibres, tissus, tissus à mailles ou corps formés, notamment des membranes.

14. Utilisation des photosensibilisateurs selon une ou plusieurs des revendications 11 à 13 pour déclencher des réactions photochimiques par action de la lumière visible.

15. Utilisation des photosensibilisateurs selon la revendication 14 pour former catalytiquement de l'oxygène singulet à partir de l'oxygène triplet par action de la lumière visible.

16. Utilisation de porphyrines liées à des polymères métallées et/ou non métallées selon la revendication 14 pour détoxiquer et/ou désinfecter de l'oxygène et notamment de l'eau ou des environnements contenant de l'eau par irradiation par de la lumière actinique.

17. Utilisation selon la revendication 14 pour le traitement des eaux usées, notamment dans les circuits d'eau ; à titre d'épuration intermédiaire ou épuration finale dans les installations de décantation industrielles ou communales, pour l'épuration de l'eau de lavage dans l'industrie des produits alimentaires, notamment lors du lavage des fruits et légumes avant la mise en conserve ; pour le maintien de la propreté des réservoirs d'eau ; pour la stabilisation de l'eau potable ; en combinaison avec des laveurs de gaz, dans lesquels il apparaît des eaux de lavage polluées ; pour le traitement de l'eau de lavage, de bain et de douche des installations industrielles et communales, notamment dans les complexes hôteliers ou les campings ; pour l'épuration des eaux de piscine, notamment en combinaison avec le réchauffement solaire de l'eau ; pour l'épuration de l'air rejeté ou de l'air de circulation, notamment de l'air des étables ou pour désodoriser les courants d'air rejeté ou l'air circulant dans les bâtiments.

18. Utilisation selon la revendication 14, **caractérisée en ce que** l'on met en oeuvre la porphyrine sous forme de structures fixées, notamment de plateaux en matériau plein ou de plateaux à revêtement ainsi que sous forme de granulés, poudres, membranes ou filtres.

19. Utilisation selon la revendication 14, **caractérisée en ce que** l'on met en oeuvre la porphyrine sous forme de réacteurs à lit plat ou de réacteurs à colonne à bulles.

20. Utilisation des dérivés de porphyrine de la formule générale I selon l'une des revendications 2 à 4, **caractérisée en ce que** l'on met en oeuvre les porphyrines pour désinfecter et/ou détoxiquer des objets de tout type.

21. Récipients ou objets pour détoxiquer et/ou désinfecter des objets, de l'eau ou des environnements contenant de l'eau, comprenant des zones superficielles en porphyrines selon l'une des revendications 2 à 4, liées à des polymères.

22. Récipients selon la revendication 21, comprenant des appareils de nettoyage pour lentilles de contact, appareils dentaires ou appareils d'orthodontie.

23. Récipients selon la revendication 21, comprenant des revêtements antibactériens de surface dans la technique médicale.
